# EUROPEAN PATENT SPECIFICATION

(11) **EP 2 295 024 B1**
(45) Date of publication and mention of the grant of the patent: **06.01.2016**
(21) Application number: 10184864.6
(22) Date of filing: 09.11.2006
(51) Int. Cl.: A61K 6/00

(54) **Denture adhesive compositions**
Gebiss-Klebezusammensetzungen
Compositions servant de ciment pour prothese dentaire

(30) Priority: 09.11.2005 US 734874 P; 09.11.2005 US 735088 P; 20.01.2006 US 760516 P; 20.01.2006 US 760528 P; 20.01.2006 US 760660 P; 31.10.2006 US 590111; 31.10.2006 US 590191; 31.10.2006 US 590225; 31.10.2006 US 590233; 31.10.2006 US 590231; 09.11.2005 US 735243 P; 09.11.2005 US 735136 P; 09.11.2005 US 735135 P; 31.10.2006 US 590232; 31.10.2006 US 590224; 20.01.2006 US 760711 P; 20.01.2006 US 760527 P; 31.10.2006 US 590145; 20.01.2006 US 760526 P
(43) Date of publication of application: 16.03.2011
(62) Divisional of application: 06827767.2
(73) Proprietor: The Procter & Gamble Company, Cincinnati, OH 45202 (US)
(72) Inventor: Rajaiah, Jayanth, Loveland, OH 45140 (US); Hamersky, Mark, William, Fairfield Township, OH 45011 (US); Smith, Steven, Daryl, Fairfield, OH 45014 (US); Leonard, Robert, Scott, Fairfield, OH 45014 (US)
(74) Representative: Joos, Uta Susanne

(56) References cited:
- WO-A-01/15657
- WO-A-01/41710
- WO-A-02/30317
- WO-A-96/25910
- WO-A-2005/081935

## Description

### TECHNICAL FIELD

This invention relates to denture adhesive compositions and in particular to improved denture adhesive compositions comprising a normalized dislodgement force of about 1100 to about 12,000 grams per sq.cm., or dislodgement force ratio from about 1.1 to about 10.

### BACKGROUND OF THE INVENTION

Ordinary removable dentures, dental plates and the like, comprise teeth mounted in a suitable plate or base. While dentures are traditionally fitted for the individual user, the fit can change over time which may result in slippage or discomfort. Denture adhesives are used to temporarily adhere the dentures to the surfaces of the oral cavity, in particular the oral mucosa. Denture adhesives are typically applied to either the denture or oral surface at the beginning of the day when the dentures are placed into the oral cavity, and the adhesives tend to bio-erode during the course of the day due to the action of saliva and chewing.

Considerable effort has been made over the years to develop improved denture adhesive products. Both synthetic and natural polymers and gums have been used alone and in combination with various adhesives and other materials in an attempt to improve hold and reduce oozing of the adhesive from under the dental plate, and to avoid messiness and difficulty of removing the residual adhesive from the mouth and dentures after use. For example, alkyl vinyl ether-maleic copolymers and salts thereof are known for providing hold in denture adhesive compositions. Such disclosures include: WO01/15657A, WO02/30317 A, WO01/41710A, WO 05/081935 A, U.S. Patent 3,003,988, Germann et al., issued October 10, 1961; U.S. Patent 4,980,391, Kumar et al., issued December 25, 1990; U.S. Patent 5,073,604, Holeva et al., issued December 17, 1991; U.S. Patent 5,525,652, Clarke, issued June 11, 1996; U.S. Patent 5,340,918, Kittrell et al., issued Aug. 23, 1994; U.S. Patent 5,830,933, Synodis et al., issued Nov. 3, 1998.

In addition to adhesion, it is desirable to reduce oozing or to reduce the negative aesthetics of oozing experienced by the consumer. Oozing may occur due to seeping of the denture adhesive from under the dental plate in the oral cavity caused by a variety of factors including a low viscosity denture adhesive, use of too much denture adhesive, improper application of the denture adhesive on the denture plate, etc. When oozing occurs in the oral cavity, the denture adhesive composition is exposed to the oral cavity. Therefore, any negative taste, negative mouth-feel, or any other any other negative aesthetic associated with the denture adhesive composition may be more noticeable and objectionable to the consumer. Sources of such negative perception may include the denture adhesive polymer itself or salts of the denture adhesive polymer, including those crosslinked with zinc salts. Taste considerations are significant since denture adhesive compositions are used in the oral cavity for up to 6-7 hours or longer. Furthermore, consumers may stop using the adhesive or may tend to apply less adhesive the next time if they experience the negative perception of ooze. This may lead to decreased denture hold or decreased denture performance. This decrease in performance can mean less denture stability, denture retention, or an increase in food lodging itself under the denture prosthesis.

In accordance with the present invention, the denture adhesive composition herein will provide these improved denture adhesive properties including improved hold, fit, ease of handling, ease of application, decreased ooze, and/or improved clean up under the varied conditions of the oral cavity.

### SUMMARY OF THE INVENTION

The present invention relates to a denture adhesive composition comprising from 10% to 90% of a denture adhesive component; from 2% to 90% of a water insoluble thermoplastic component selected from microcrystalline wax and mixtures thereof with Polyethylene; wherein the composition has a normalized dislodgement force of from 1100 to 12,000 grams per sq.cm, or a dislodgement force ratio from 1.1 to 10.

### BRIEF DESCRIPTION OF THE DRAWINGS

While the specification concludes with claims particularly pointing out and distinctly claiming the invention, it is believed that the present invention will be better understood from the following description taken in conjunction with the accompanying drawings in which:
Fig. 1 is a top plain view of an embodiment of a concave shaped denture adhesive article having symmetrical dimensions;
Fig. 2 is a top plain view of an embodiment of a concave shaped denture adhesive article having asymmetrical dimensions.

### DETAILED DESCRIPTION OF THE INVENTION

A detailed description of essential and optional components of the present invention is given below.

### DEFINITIONS

The abbreviation "cm", as used herein, means centimeter. The abbreviation "mm" as used herein, means millimeter. The abbreviation "g" as used herein, means gram.

The term "denture" and/or "denture prosthesis" as used herein refers to either the upper or lower denture, or both.

The term "denture adhesive article" and/or "article" as used herein refers to articles designed to fit, conform and adhere to contoured surfaces, such as a denture, as well as the gums or the roof of the mouth. The articles herein are substantially solid prior to use and can be picked up manually in substantially one piece and positioned on the denture.

The term "flexible" or "flexible article" as used herein means that a 0.67 mm thick piece of the article may be wrapped 180 degrees around a solid cylinder of 1 cm diameter without cracking upon visual observation.

The term "safe and effective adhesive amounts" as used herein means an amount sufficient to provide adherence to the oral cavity and/or provide adherence of a denture to the oral cavity, without toxicity to the user or damage to oral tissue.

By "safe and effective amount", as used herein, is meant an amount of an agent high enough to significantly (positively) modify the condition to be treated or positively modify the benefit sought, but low enough to avoid serious side effects (at a reasonable benefit/risk ratio), within the scope of sound medical/dental judgment. The safe and effective amount of an agent may vary with the particular condition being treated, the age and physical condition of the patient being treated, the severity of the condition, the duration of treatment, the nature of concurrent therapy, the specific form of the source employed, and the particular vehicle from which the agent is applied.

The term "AVE/MA" as used herein refers to alkyl vinyl ether-maleic acid or anhydride copolymer. The term "mixed polymer salts" or "mixed salts", as used herein, refers to salts of AVE/MA where at least 2 different cations are mixed on the same polymer with each other or with other salts.

The term "free acid" or "FA" component, as used herein, refers either to the unreacted carboxyl groups (-COOH) of AVE/MA copolymer plus any other monovalent cations of carboxyl groups (e.g., COONa) of the polymer. Monovalent cations include Group IA cations, such as sodium, potassium, hydrogen, etc. In one embodiment, the term "free acid" refers to the unreacted carboxyl groups (-COOH) of AVE/MA plus sodium and potassium cations. In another embodiment, the term "free acid" refers only to the unreacted carboxyl groups (-COOH) of the AVE/MA.

The term "toxicologically-acceptable", as used herein, is used to describe materials that are suitable in their toxicity profile for administration to humans and/or animals.

By "non-aqueous", as used herein, is meant that the composition does not contain added water but may contain water that is included in another component as supplied commercially by the manufacturer.

The term "water-insoluble" as used herein refers to a material that, when exposed to an excess of water, does not dissolve, but may disperse to varying degrees. In some embodiments the term "water-insoluble" refers to a material that is less than 10%, 5%, 2%, or 1% soluble in water.

The term "thermoplastic" as used herein refers to a material that melts, softens, and becomes more flexible, extrudable, deformable, shapable, moldable, flowable, processable, and/or changes rheology when exposed to heat. In one embodiment the material generally solidifies, hardens, and/or substantially returns to its original condition, when subsequently cooled.

The term "bioerodible" as used herein means that the composition, when exposed to excess of water or saliva, will erode over time due to physical and/or chemical action. The time necessary to erode the composition can be any length of time from instantaneous to five days; in one embodiment the time to erode is from 1 to 3 days. The composition may erode completely or substantially, however ultimately the composition will lose its original form and/or integrity. For example, after application and use for at least 24 hours in the oral cavity the composition will not have sufficient product integrity to easily separate or peel, in its original form, from the denture or oral surface. In one embodiment the composition bioerodes such that no portion of the composition remains on the denture or mouth after the composition has been used in the oral cavity for about 24 hours. In another embodiment some portion or residue from the composition remains on the denture or oral surface after removing the denture from the oral cavity; however, this portion or residue from the composition can be cleaned by brushing away with a toothbrush, but not easily separated from the denture.

The percentages used herein to describe the cationic salt function of the alkyl vinyl ether-maleic acid or anhydride copolymers are defined as the stoichiometric percent of the total initial carboxyl groups reacted on the polymer.

All other percentages used herein are by weight of the composition unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

### DENTURE ADHESIVE COMPOSITION

The present invention relates to denture adhesive compositions designed to fit, conform and adhere to contoured surfaces such as a denture as well as the gums or the roof of the mouth.

In one embodiment the compositions described herein are selected from the group consisting of creams, pastes, gels, liquids, and mixtures thereof. In another embodiment the compositions can be extruded out of a nozzle of a container such as a tube, pump and/or syringe. In yet another embodiment the compositions herein are articles. In one embodiment the articles herein minimize or avoid the problem of premature sticking during application of the article to the denture. That is, with some prior art denture adhesive articles, before the article can be properly positioned over a target surface on the denture, inadvertent contact of the article with the denture may cause premature sticking at one or more locations on the denture. This may inhibit proper positioning of the article. Premature sticking may also cause contamination or degradation of the article prior to final positioning on the denture.

In one embodiment the term "dry tack" as used herein means that present articles exhibit minimal and/or no adhesive or cling properties in the dry state until activated by pressure applied by a user. In one embodiment this characteristic permits the present articles to be stored and dispensed in any desired mode without encountering the difficulties of premature clinging or adhering to themselves, and without the need for separate release sheets, liners, spacers, or the like. At the same time, in one embodiment when pressure activated at the desired location and at the desired time, the articles can, in the dry state, exhibit sufficient adhesive properties to form a bond to most plastic surfaces including a denture surface, this bond being sufficiently strong to survive handling of the denture without bond failure. Therefore, in one embodiment the articles herein, in the dry state, adhere to a target denture surface only when pressed thereagainst, thereby minimizing or avoiding this problem of inadvertent adherence during positioning on the denture surface. In one embodiment then, the articles herein do not have to be moistened or wet prior to application to the denture, thus providing a simple and easy way to apply an article to the denture.

In one embodiment the term "dry tack" as used herein means that present articles exhibit minimal and/or no adhesive or cling properties until activated by pressure applied by the user after the article has been warmed by the hands of the user, potentially during the course of application of the article to the denture surface.

In another embodiment the articles herein are non-tacky to the touch prior to application to the denture.

In another embodiment the term "dry tack" as used herein means articles herein in a dry and unwetted state, are capable of immediate bonding by surface attachment to a dry plastic, polymethyl methacrylate, and/or other denture prothesis substrate, upon subjecting the article to pressure. In one embodiment the dry article, develops bonding by surface attachment to a dry denture prosthesis substrate upon the application of finger pressure whereby the article remains bonded under its own weight, and the article herein will not remain bonded to this dry substrate under its own weight without using finger pressure to apply the article to the substrate. In one embodiment the force or pressure may be generated by one or more fingers. This force or finger pressure, in one embodiment, may be applied for 1-10 seconds or longer. In another embodiment the bonding of the article to the substrate is maintained from about 10 seconds to about 3 minutes or longer, in another embodiment from about 30 seconds to about 1 minute or longer.

In one embodiment the dry tack of the article is from 0.025, 0.1, 1, 10, 100, 1000 gram force/square centimeter to 10, 100, 1000, 10,000, 50,000, 100,000 gram force/square centimeter and any combination thereof.

In one embodiment the dry tack of an article that can be repositioned is from 0.025 grams/force square centimeter to 0.30 grams/force square centimeter, and in another embodiment from 0.025 gram force/square centimeter to about 0.25 gram force/square centimeter.

It is reported that the room temperature modulus of any tacky adhesive is less than 3 x 10⁶ dynes/cm² when measured at a frequency of 1 Hz. This finding is a criterion for tack and has been given the name "Dahlquist criterion for tack."(Adhesion and Adhesives Technology, by Alphonsus Pocius, 2nd Edition, 2002 Carl Hanser Verlag, Munich).

In one embodiment of the current invention, the article has a modulus greater than the 'Dahlquist criterion for tack' of about 3 x 10⁶ dynes/cm². In another embodiment, the article has a shear storage modulus G' (measured in dynes/cm² at a frequency of 1 Hz at 25°C) greater than 5 x 10⁶; in another embodiment greater than 1 x 10⁷; in another embodiment greater than 5 x 10⁷ ; and in another embodiment greater than 8 x 10⁷.

In one embodiment the composition has a shear storage modulus G' (measured in dynes/cm² at a frequency of 1 Hz at 25°C) from about 1 x 10⁶, 3 x 10⁶, 5 x 10⁶, 1 x 10⁷ , 5 x 10⁷, and 8 x 10⁷ to about 5x 10⁸, 5 x 10⁷, 1 x 10⁸, 5 x 10⁹, 1 x 10⁹, and 1 x 10¹⁰ and/or any combination thereof.

In one embodiment the composition is an article and has a flexural stiffness of less than about 10 grams/cm, in another embodiment less that about 5 grams/cm, in another embodiment less that about 3 grams/cm, in another embodiment less than 2 grams/cm and in yet another embodiment from 0.1, 0.5, 1, to 2, 3, 5, 10 grams/cm, in any combination, flexural stiffness as measured on a Handle-O-Meter, model #211-300, available from Thwing-Albert Instrument Company of Philadelphia, PA as per test method ASTM D2923-95.

In one embodiment the compositions herein have a normalized dislodgement force of from 1100 to 12,000 grams per sq.cm, in another embodiment from 1300 to 10,000 grams per sq.cm, in another embodiment from 1200 to 5000 grams per sq.cm, in another embodiment from 1400 to 5000 grams per sq.cm, in another embodiment from 1300 to 2500 grams per sq.cm, in another embodiment from 1750 to 2500 grams per sq.com. In another embodiment the normalized dislodgement force is from 1100, 1200, 1300, 1400, 1500, 1750 grams per sq.cm. to 12,000, 10,000, 7500, 5000, 2500, 2250 grams per sq.cm, and/or any combination thereof. In one embodiment the dislodgement force ratio is from 1.1, 1.2, 1.3, 1.4, 1.5, 1.6, 1.7, 1.8, 1.9, 2.0 to 1.3, 1.4, 1.5, 1.6, 1.7, 1.8, 1.9, 2.0, 3, 4, 5, 6, 8, 10, and/or any combination thereof. In one embodiment the dislodgement force ratio is from 1.1 to 10, from 1.1 to 8, from 1.3 to 4, and/or from 1.3 to 2.5.

In one embodiment the articles herein are substantially solid prior to use and can be picked up manually, in substantially one piece, and positioned on the denture. Additionally, in one embodiment the articles are capable of being picked up manually, and positioned on the denture, resulting in little or no residue on the fingers. In another embodiment the articles comprise a single layer. In yet another embodiment the articles are laminates, and/or composites. In one embodiment the articles are pre-shaped and/or preformed. In another embodiment the articles herein may be dispensed by the consumer via a unit dose package, multi-dose package, pump, sachet, syringe, or tube, and shaped by the consumer; and in yet another embodiment the articles may be shaped by the consumer without leaving a substantial residue on their hands.

In one embodiment, the denture adhesive article is sufficiently flow-able to allow it to be applied from a tube and subsequently picked up and positioned on the denture. In one embodiment, the denture adhesive article is sufficiently flow-able to allow it to be applied from a tube and subsequently picked up, and positioned on the denture, with little/no residue on the fingers. In another embodiment the denture adhesive article comprises a solvent which results in an article that is sufficiently flow-able to be applied from a tube. The solvent may be subsequently dissipated, by evaporation, bio-absorption, dispersion, dissolution, etc. In another embodiment the above mentioned optional solvent is also miscible with the water insoluble component. In one embodiment, the denture adhesive composition is sufficiently flow-able to allow it to be applied from a tube and only minimally ooze out from under the denture. In one embodiment the denture adhesive composition has a "normalized ooze amount" from 0%, 0.00001%, 0.001%, 5%, 10%, 15%, 20%, 25%, 30% to 15%, 20%, 25%, 30%, 40%, 50% and/or any combination thereof. In one embodiment the denture adhesive composition has an "ooze ratio" from 0, 0.00001, 0.001, 0.01, 0.1, 0.2, 0.3, 0.4, 0.5, 0.6 to 0.3, 0.4, 0.5, 0.6, 0.7, and/or any combination thereof.

Regardless of the form of dispensing the article, including but not limited to pre-dosed ready to use articles and/or articles which are dispensed such as from a tube, the articles are substantially solid prior to use and can be picked up manually. Denture adhesive articles that can be dispensed from a tube can be identified as articles by the following method (pPlease read all steps before starting test.)
1. Fill product into a tube with a 0.16" diameter nozzle.
2. Extrude a 1" long strip of product onto a denture tile (1.5" x 1.5" square tile made from denture-plastic) taking care to hold the nozzle about 1/8" above the denture-tile. Do not touch the nozzle to denture-tile while extruding the product.
3. After about 1" of product has been extruded, hold nozzle about 1/8" above the denture-tile and use a spatula to cut the strip against the nozzle. Do not touch or smear the nozzle against the denture-tile while cutting strip.
4. Use thumb and forefinger to hold the middle of the strip and pick it up vertically off the denture-tile. Do not use a wiping motion of the fingers against the denture-tile.
5. The composition is an article if it can be picked up in substantially one piece.

Some denture adhesive articles are pre-dosed and/or ready to use. A user may be able to identify these items visually as a denture adhesive article, as they are often in the form of a strip contained within a package. However, if not evident that these denture adhesive products are articles, these denture adhesive articles can be identified as articles by the following method:
1. Shape composition into a sheet about 0.67 mm thick x 8 mm wide x 44 mm long.
2. Place sheet on a denture-tile.
3. Use fingers to pick up sheet.
4. The composition is an article if it can be picked up in substantially one piece.

Substantially in one piece means, as used herein, that from 75, 80, 85, 90% to 100, 90, 85, 80, 75, 70% and/or any combination thereof of the denture adhesive composition remains in one piece when manually picked up from the denture surface.

In addition to the aforementioned test methods denture adhesive articles can also be identified as articles by the amount of ooze, as determined by the ooze method (as defined herein). In addition to being able to be manually picked up and moved in substantially one piece, a denture adhesive article has a normalized ooze amount of from 0, 3, 5, 10, 15, 20, 25% of the total composition to 30, 25, 20, 15, 10, 5, 3 % of the total composition and/or any combination thereof and/or the ooze ratio is from 0, 00001, 0.001, 0.01, 0.1, 0.2, 0.25, 0.3, to 0.1, 0.2, 0.25, 0.3, 0.4, 0.5 and/or any combination thereof.

In one embodiment the composition herein comprises ingredients of natural origin.

In one embodiment the composition herein comprises a homogeneous mixture of the denture adhesive component and the water insoluble thermo-plastic component.

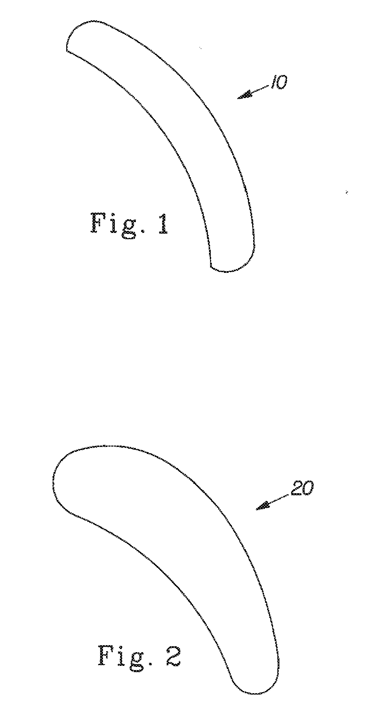

The denture adhesive article can have a variety of shapes and sizes including but not limited to a concave shape which is either symmetrical or asymmetrical. Fig. 1 shows a symmetrical concave shaped denture adhesive article 10. Fig. 2 shows an asymmetrical concave shaped denture adhesive article 20, in particular Fig. 2 shows a kidney shaped denture adhesive article 20.

### DENTURE ADHESIVE COMPONENT

The present invention comprises a safe and effective adhesive amount of a denture adhesive component, i,e. at a level of from 10% to 90%, in another embodiment from 15% to 70%, in another embodiment from 20% to 70%, in yet and in another embodiment from 25% to 65%, and in yet another embodiment from 30% to 65%, by weight of the composition. In one embodiment the composition of the present invention comprise from at least 20 percent by weight, and in another embodiment at least 30 percent by weight of the composition, of a denture adhesive component.

In one embodiment the denture adhesive components herein are mucoadhesive, hydrophilic, water soluble, have the property of swelling upon exposure to moisture, and/or to form a mucilaginous mass when combined with moisture. In one embodiment the denture adhesive components are selected from the group consisting of natural gums, synthetic polymeric gums, AVE/MA, salts of AVE/MA, AVE/MA/IB, salts of AVE/MA/IB, copolymer of maleic acid or anhydride and ethylene and salts thereof, copolymer of maleic acid or anhydride and styrene and salts thereof, copolymer of maleic acid or anhydride and isobutylene and salts thereof, polyacrylic acid and polyacrylates thereof, polyitaconic acid and salts thereof, mucoadhesive polymers, water-soluble hydrophilic colloids, saccharide derivatives, cellulose derivatives, and mixtures thereof. Examples of such materials include karaya gum, guar gum, gelatin, algin, sodium alginate, tragacanth, chitosan, acrylamide polymers, carbopol, polyvinyl alcohol, polyamines, polyquarternary compounds, polyvinylpyrrolidone, cationic polyacrylamide polymers, AVE/MA, AVE/MA/IB, mixed salts of AVE/MA, mixed salts of AVE/MA/IB, polymeric acids, polymeric salts, polyhydroxy compounds, and mixtures thereof.

In one embodiment the denture adhesive components are selected from the group consisting of salts of AVE/MA, mixed salts of AVE/MA, cellulose derivatives (such as methylcellulose, carboxymethylcellulose, hydroxyethylcellulose, hydroxypropylcellulose, hydroxy-propylmethylcellulose, and mixtures thereof), polyethylene glycol, karaya gum, sodium alginate, chitosan, corn starch, and mixtures thereof. In yet another embodiment, the adhesive component is selected from the group consisting of mixed salts of AVE/MA, cellulose derivatives, and mixtures thereof.

In one embodiment the denture adhesive component is not thermoplastic and/or comprises only low levels of water soluble thermoplastic polymers, from 0.01 to 5% of water soluble thermo-plastic polymer such as polyethylene oxide, hydroxypropyl cellulose, hydroxypropylmethylcellulose; polyethylene glycol; in another embodiment from 0.01 to 1% of water soluble thermoplastic polymer, or is essentially free of water soluble thermoplastic polymers.

### Alkyl Vinyl Ether-Maleic Copolymer

In one embodiment of the invention the denture adhesive component is AVE/MA or salts of AVE/MA. The alkyl vinyl ether-maleic acid co-polymer comprises or consists essentially of the repeated structural unit: 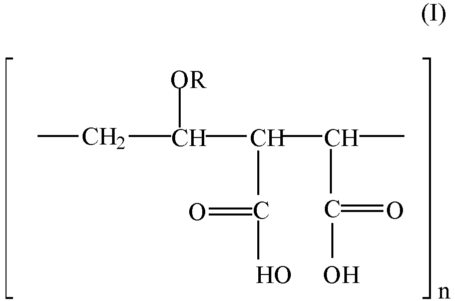 wherein R represents an alkyl radical, in one embodiment a C₁ to C₅ alkyl radical, n is an integer greater than one representing the number of repeated occurrences of the structural unit in a molecule of the polymer.

In one embodiment, the adhesive component is AVE/MA and salts thereof, preferably mixed salts of AVE/MA, wherein the copolymer contains a cationic salt function comprising a cation selected from Group IA and Group 2A cations of the periodic table, yttrium, titanium, zirconium, vanadium, chromium, manganese, iron, nickel, copper, zinc, boron, aluminum, and mixtures thereof. In another embodiment, the adhesive component is a mixed salt of AVE/MA containing a cationic salt function comprising a cation selected from strontium, zinc, iron, boron, aluminum, vanadium, chromium, manganese, nickel, copper, yttrium, titanium, magnesium, calcium, sodium, and mixtures thereof, and in yet another embodiment the cation is selected from strontium, zinc, iron, magnesium, calcium, sodium, and mixtures thereof.

AVE/MA contains, in one embodiment, a cationic salt function comprising from 5% to 50%, in another embodiment, from 10% to 40%, in yet another embodiment, from 10% to 35% (of the total initial carboxyl groups reacted) zinc cations. These zinc cations can be mixed with other cations selected from the group consisting of: from 5% to 65%, preferably from 10% to 60%, strontium cations, from 0.001% to 2.5%, preferably from 0.01% to 2% of iron, boron, aluminum, vanadium, chromium, manganese, nickel, copper, yttrium, and/or titanium cations, from 5% to 65%, preferably from 15% to 50% of calcium and/or magnesium cations and/or sodium cations. AVE/MA and salts thereof, are also described in U.S. Patent Nos. 5,073,604 to Holeva et al., issued 12/17/91; 5,525,652, issued Jun. 11, 1996, Clarke et al.; 6,025,411, issued Feb. 15, 2000, Wong et. al.; 4,758,630, issued July 19, 1988, Shah et al.; 5,304, 616, issued April 19, 1994, Rajaiah et al.; 5,424,058, issued June 13, 1995, Rajaiah; 5,424,058, issued 6/13/95, Rajaiah et al.; 4,758,630, issued July 19, 1988, Shah et al.; 5,830,933, issued Nov. 3, 1998, Synodis et al.; 2,047,398, issued July 14, 1936, Voss et al.; 3,003,988, issued Oct. 10, 1961, Germann et al.; 5,880,172, Rajaiah et al., issued March 9, 1999; 5,900,470, Prosise et al., issued 5/4/99; 5,037,924, Tazi et al., issued 8/6/91; 5,082,913, Tazi et al, issued 1/21/92. Salts of AVE/MA are also described in U.S. Patent Nos. 6,355,706 to Rajaiah, et al., issued March 12, 2002; 6,617,374 to Rajaiah, et al., issued September 9, 2003.

In one embodiment the free acid level of the salts of the AVE/MA or AVE/MA/IB is at least 36%, in another embodiment is from 36% to 60%, and even in another embodiment is from 40% to 55%, of the total initial carboxyl groups of the copolymer.

In one embodiment the specific viscosity of the starting copolymer acid or copolymer anhydride is from 1.2 to 14, when preferably measured in a 1% weight/volume solution in MEK (methyl ethyl ketone) at 25°C. Other methods and solvents can be used to measure the specific viscosity such as a 1% weight/volume solution in DMF (dimethyl formamide) at 25°C and a 1% weight/volume solution in 2-butanone at 25°C.

Suitable AVE/MA copolymers may be prepared by well-known methods of the prior art; see, for example, US 2,782,182, and US 2,047,398.

Methods of making mixed salts of AVE/MA polymers are further disclosed in U.S. Patent Nos. 5,073,604, Holeva et al., issued Dec. 17, 1991 and 5,872,161, Liang et al., issued Feb. 16, 1999.

### WATER INSOLUBLE COMPONENT

The present composition comprises a safe and effective amount of a water insoluble thermoplastic component. In one embodiment this component is at a level from 2, 5, 10, 20, 25, 30, 35% to 45, 50, 60, 70, 90%, and/or any combination thereof to create ranges, by weight of the composition , e.g. the water insoluble component level is from 10% to 90%, from 20% to 70%, from 25% to 60%, or from 35% to 60% by weight of the composition. In yet another embodiment the water insoluble component is both water insoluble and substantially non-swellable in water.

In one embodiment the water insoluble components selected from microcrystalline wax, polyethylene and mixtures thereof. In one embodiment the water insoluble thermoplastic component is selected from microcrystalline wax and mixtures thereof with polyethylene.

In one embodiment the composition herein has a water insoluble thermoplastic component having a penetration of 25 to 350; in another embodiment from 27 to 250 in another embodiment from 30 to 150, and in another embodiment from 40 to 150, and in another embodiment from 50 to 80, and in yet another embodiment from 60 to 80. The penetration values are from 25 to 250 when measured using the ASTM D1321 method and the penetration values are from 25 to 350 when measured using the ASTM D937 method, both are existing methods known in the art. Although ASTM D937 and ASTM D1321 generally are used to measure the penetration of petrolatum or petroleum waxes, respectively, these methods may be used to measure the penetration of waxes derived from petroleum and other types of water insoluble thermoplastic components with appropriate modifications, for example, these other components may need to be melted at higher temperature which will be apparent to one of skill in the art.

In obtaining the above penetration values, water insoluble thermoplastic component that are outside of these penetration values may be mixed with another ingredient modifies the penetration of the thermoplastic component. Therefore the water insoluble thermoplastic component may be a single ingredient or may be a mixture of ingredients. For example, Multiwax W 180 manufactured by Witco (Crompton, Sonneborn), having a penetration value of 15 to 20 may be mixed with petrolatum (at a 1:1 ratio) to raise the penetration value to above 25. In one embodiment the water insoluble thermoplastic component is synthetic microcrystalline wax.

In another embodiment the wax is microcrystalline wax manufactured by Crompton, Sonneborn (Witco) and referred to and sold under the trademark MutiwaxW-835. This wax has a melt point ranging from about 73.9 °C to about 79.4 °C (ASTM D127), has a penetration at 25°C of from 60 to 80 (ASTM D1321), has a kinematic viscosity at 98.9°C, of from 75 to 90 (ASTM D2161), has a flash point, COC, of about 246°C Min. (ASTM D92), and has a congealing point, of about 77°C (ASTM D938).

In one embodiment the microcrystalline wax useful herein can have a melting point of from 65°C to 90°C, in another embodiment can have a melting point of from 80°C to 90°C.t

In another embodiment the water insoluble thermoplastic component is polyethylene such as A-C 1702 and A-C 6702 made by Honeywell, with a penetration value of 98.5 and 90.0, respectively, under ASTM D1321.

In one embodiment if the composition contains polyethylene oxide, then either the water insoluble component is thermoplastic or the article may not include a fibrous paper web or paper laminate.

In one embodiment the composition herein is substantially free of honey mixed with alcohol. In another embodiment the article is substantially free of polyvinyl acetate resin in ethyl alcohol.

### MISCELLANEOUS OPTIONAL INGREDIENTS

### Plasticizing Agent

The compositions of the present invention may also optionally comprise a safe and effective amount of one or more toxicologically-acceptable plasticizers. In one embodiment the level of the plasticizing agent ranges from 0.0% to 40%, in one embodiment from 0.01% to 40%, in another embodiment from 1% to 10%, in another embodiment from 2% to 5%, by weight of the composition. In yet another embodiment the denture adhesive composition does not comprise a plasticizer.

Suitable plasticizing agents of the present invention include, but are not limited to, polyols (such as sorbitol); glycerin; propylene glycol; acetylated monoglyceride; hydrogenated starch hydrolysates; corn syrups; and derivatives thereof; xylitol, glycerol monoesters with fatty acids; triacetin; diacetin; and monoacetin; dimethyl phthalate; diethyl phthalate; dioctyl phthalate; diethylene glycol; triethylene glycol; tricresyl phosphate; dimethyl sebacate; ethyl glycolate; ethyl-phthalyl ethyl glycolate; o- and p-toluene ethyl sulfonamide; phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof. In another embodiment the plasticizer is water insoluble.

In one embodiment, the denture adhesive composition, when extruded thermoplastically, does not cure and set as a result of the action of the plasticizer component. In another embodiment the plasticizer component does not solidify the water insoluble component or the denture adhesive composition. In another embodiment the water insoluble thermoplastic component does not cure and set.

Alternatively, in one embodiment the denture adhesive composition may be substantially free of plasticizers. In one embodiment the denture adhesive composition can be substantially free of polyethylmethacrylate, triacetin, phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

### Gellant Agents

The compositions of the present invention may also optionally comprise a safe and effective amount of one or more toxicologically-acceptable gellants. In one embodiment the level of the gellant agent ranges from 0.01 % to 40%, in another embodiment from 1% to 10%, in another embodiment from 2% to 5%, by weight of the composition.

Suitable gellant agents of the present invention include, but are not limited to, polyvinylpyrrolidone/eicosene copolymer sold under the tradename Ganex V-220F from ISP; tricontanyl polyvinylpyrrolidone sold under the tradename Ganex WP-660 from ISP; polyamide gallants including Sylvaclear, Sylvacote, Sylvagel, Uniclear all available from Arizona Chemical including Sylvaclear Lightwax; Sylvaclear PA 20; Sylvaclear PA 30; Sylvaclear PA 50; Sylvacote 2228; Sylvacote 2228E; Sylvagel 5000; Sylvagel 6000; Uniclear 100; Uniclear 100VG; Uniclear 80; Uniclear 80V; and mixtures thereof.

### Flavors, Fragrance, Sensates

The compositions of the present invention may also include one or more components which provide flavor, fragrance, and/or sensate benefit (warming or cooling agents). Suitable components include menthol, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof, as well as coolants. The coolant can be any of a wide variety of materials. Included among such materials are carboxamides, menthol, ketals, diols, and mixtures thereof. In one embodiment the coolants in the present compositions are selected from the group consisting of the p-menthane carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979. These agents may be present at a level of from 0% to 40%, in another embodiment from 0.05 to 5%, and in another embodiment from 0.1 to 2%, by weight of the composition.

### Other Optional Ingredients

The denture adhesive compositions may also comprise one or more therapeutic actives suitable for topical administration. Therapeutic actives may be present at a level of from 0% to 70%, by weight, and in one embodiment from 1% to 20% by weight. Therapeutic actives include antimicrobial agents such as iodine, triclosan, peroxides, sulfonamides, bisbiguanides, or phenolics; antibiotics such as tetracycline, neomycin, kanamycin, metronidazole, cetylpyridium chloride, domiphen bromide, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannous fluoride, MFP; anesthetic agents such as lidocaine or benzocaine; anti-fungals such as those for the treatment of candida albicans; aromatics such as camphor, eucalyptus oil, and aldehyde derivatives such as benzaldehyde; insulin; steroids; herbal and other plant derived remedies; and baking soda. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

Other suitable ingredients include colorants, preservatives (such as methyl and propyl parabens), thickeners such as silicon dioxide, and polyethylene glycol. Colorants, preservatives, thickeners may be present at levels of from 0% to 20%, by weight of the composition, in another embodiment from 0.1% to 10%, by weight.

Additionally, the compositions may also comprise one or more solvents. These optional solvents may be miscible with the water insoluble component and/or be capable of being dissipated in-situ. In one embodiment these solvents may be dissipated in-situ by evaporation, dissolution, dispersion, bio-absorption, or any other suitable means. In another embodiment these solvents may be dissipated in-situ to leave behind a denture adhesive composition. Such solvents may include materials with a viscosity ranging from 0.01, 0.1, 1, 5 centipoise at 20°C, to 5, 10, 100, 1000 centipoise at 20°C in any combination of these levels. In one embodiment these solvents may be silicones, hydrocarbons, iso-dodecane, iso-hexadecane, iso-eicosane, and/or polyisobutene. Suitable grades of solvents include the Permethyl series (sold by Prespers Inc., New Jersey) such as Permethyl 97A, 99A, 101A, 102A, and mixtures thereof.

### PROCESS FOR PREPARATION OF THE COMPOSITION

In one embodiment the composition is an article (not part of the invention) formed by processes conventional in the arts, e.g. the film making industries such as casting, coating, calendaring, and extrusion. In one embodiment the separate components of the article are melted and then blended in a mixing tank until a homogeneous mixture is achieved. Thereafter, the melted mixture may be cast to an acceptable thickness, on an appropriate substrate. Examples of such substrates include Mylar, continuous moving stainless steel belt (which may eventually entering a dryer section if needed), release paper and the like. The articles are then cooled. The articles may then be dried if needed, e.g. in a forced-air oven. The temperature of the drying air and length of drying time depend on the nature of the solvent utilized as is recognized in the art. Generally, the drying temperatures include a temperature between 25°C and 140°C, in another embodiment from 60° and 90° C for a duration of 20 minutes to 60 minutes, in another embodiment from 30 to 40 minutes. The article may then be cut into desired shapes with desired dimensions and then stacked and/or subsequently packaged.

In one embodiment, after processing, the article is then die-cut into desired shapes. These shapes may facilitate application of the article to the dentures.

In one embodiment in particular the present article is processed as follows: 1. melt the wax component; 2. mix the AVE/MA salt(s) with any other adhesive component; 3. add the adhesive mixture to the wax melt; 4. stir to made a homogeneous mixture; 5. pour the homogeneous mixture into mold or onto a suitable surface; 6. cool the mixture until it solidifies; 7. remove from substrate or mold or cut into the desired shape.

Another conventional film-making process known in the art is extrusion. This method is possible with films wherein the film forming ingredient comprises a variety of extrudable materials. The mechanical particulars of the extrusion process, e.g. the particular equipment utilized, the extruding force, the shape and temperature of the orifice and/or dies are considered to be within the skill of the art and can be varied in a known manner to achieve the physical characteristics of the articles described herein.

In one embodiment the thickness of the articles herein is generally between 0.1 mm to 2.5 mm, in another embodiment is from 0.4 mm to 1.5 mm thick, in another embodiment is from 0.5 mm to 1mm thick. The article may be thicker or thinner depending on the degree of cushioning desired by the user or wearer.

In one embodiment the compositions herein may optionally be multiphase or have visually distinct phases. In another embodiment the articles herein may optionally have a release liner. In another embodiment the composition is a cream, paste, liquid and/or gel formed by known processes. These include adding the denture adhesive components into the liquid and/or thermoplastic components and mixing with heat until a uniform product forms.

### COMPOSITION USE

The present compositions are generally applied to the denture prosthesis and thereafter the denture is secured to the oral cavity. In one embodiment the dentures are dried prior to application of the composition. In one embodiment it is not necessary to wet the composition and/or the denture prosthesis prior to applying it to the denture prosthesis in order to make the composition stick to the denture prosthesis. The composition may be applied to any suitable location on the prosthesis. In one embodiment the denture wearer generally wears the composition from 1 hour to 3 days, in another embodiment from 6 hours to 24 hours. After usage the prosthesis is removed from the oral cavity, and any remaining composition may be cleaned from the prosthesis, for example by gentle scrubbing with water and a brush.

The following examples further describe and demonstrate embodiments within the scope of the present invention. The examples are given solely for the purpose of illustration and are not to be construed as limitations of the present invention.

### EXAMPLES

### EXAMPLE I

| | A | B | C | D | E | F | G | H |
|---|---|---|---|---|---|---|---|---|
| | Grams | Grams | Grams | Grams | Grams | Grams | Grams | Grams |
| Ca/Zn AVE/MA Salt | 33.00 | 33.00 | 33.00 | 53.00 | | 33.00 | 28 | 24.5 |
| CMC | 20.00 | 20.00 | 20.00 | | 53.00 | 25.00 | 15 | 28.5 |
| AVE/MA Acid S-97 | | | 1.00 | | | | | |
| Microcrystalline Wax¹ W-835 | 46.92 | 47.00 | 47.00 | 47.00 | 47.00 | 42.00 | 45.52 | 46.92 |
| Flavors | | | 0.50 | | | | 0.4 | |
| Sacharrin | 00.08 | | 0.16 | | | | 0.08 | 0.08 |
| Colorants | | | 0.10 | | | | | |
| Silica | | | | | | | 1 | |
| Corn Starch | | | | | | | 10 | |

| | | | | | | | | |
|---|---|---|---|---|---|---|---|---|
| ¹ Multiwax W 835 manufactured by Witco (Crompton. Sonneborn). The Multiwax W 835 may also be substituted with either Polyethylene A-C 1702 or Polyethylene A-C 6702. | | | | | | | | |

The Microcrystalline Wax W-835 (or Polyethylene AC 1702 or A-C 6702) is melted, and the other ingredients are blended with it. The mixture is then made into sheets by any suitable means such as extrusion or rolling into sheets of suitable thickness such as 0.25mm, 0.45mm, 0.50mm, 0.67mm, 0.73mm, or 1.0mm. The sheet is then cut into shapes suitable for application to dentures.

In Example I all or part of the Ca/Zn AVE/MA salt may be substituted with Mg/Zn/Na AVE/MA Salts and/or Ca/Na AVE/MA salts; all or part of the CMC may be substituted with caraggeenan, and/or suitable cellulose derivatives; parts of the Microcystalline Wax W-835 may be substituted with Polyethylene A-C 1702 (available from Honeywell), and/or Polyethylene A-C 6702 (available from Honeywell); and/or, the amount of each ingredient may also be increased or decreased by up to about 50%. Each of the above examples may be blended with each other prior to making into articles; and/or used in conjunction with each other to form multilayer articles.

In Example II and III comparative Examples are shown.

### EXAMPLE II

| | A | B | C | D | E | F |
|---|---|---|---|---|---|---|
| | Grams | Grams | Grams | Grams | Grams | Grams |
| Ca/Zn AVE/MA Salt | 33.00 | 33.00 | 33.00 | 53.00 | | 20.00 |
| CMC | 20.00 | 20.00 | 20.00 | | 53.00 | 33.00 |
| Mineral Oil | 17.96 | 11.98 | 5.99 | 17.96 | 11.98 | 5.99 |
| Microcrystalline Wax² W-835 | 11.73 | 23.46 | 35.19 | 11.75 | 23.50 | 35.25 |
| Flavors | | | | | | 0.5 |
| Sacharrin | 0.02 | 0.04 | 0.06 | | | 0.08 |
| Colorants | | | | | | 0.10 |
| Silica | 0.86 | 0.57 | 0.29 | 0.86 | 0.57 | 0.29 |
| Petrolatum | 16.43 | 10.96 | 5.48 | 16.43 | 10.96 | 5.48 |

| | | | | | | |
|---|---|---|---|---|---|---|
| ² Multiwax W 835 manufactured by Witco (Crompton, Sonneborn). The Multiwax W 835 may also be substituted with either Polyethylene A-C 1702 or Polyethylene A-C 6702. | | | | | | |

In Example II, the Microcrystalline Wax W-835 (or Polyethylene AC 1702 or A-C 6702) and Petrolatum are melted, and the other ingredients are blended with it. All or part of the Ca/Zn AVE/MA salt may be substituted with Mg/Zn/Na AVE/MA Salts and/or Ca/Na AVE/MA salts; all or part of the CMC may be substituted with caraggeenan, and/or suitable cellulose derivatives; all or part of the Microcystalline Wax W-835 may be substituted with Polyethylene A-C 1702 (available from Honeywell), and/or Polyethylene A-C 6702 (available from Honeywell); and/or, the amount of each ingredient may also be increased or decreased by up to about 50%. Each of the above examples may be blended with each other.

### Example III

| | A | B | C | D | E* |
|---|---|---|---|---|---|
| | % | % | % | % | % |
| Ca/Zn AVE/MA Salt | 33 | 33 | 33 | 33 | 33 |
| CMC | 20 | 20 | 20 | 20 | 20 |
| Silica | 1.03 | 0.97 | 0.86 | 1.08 | 1.14 |
| Mineral Oil | 21.56 | 20.36 | 17.96 | 22.75 | 23.95 |
| Petrolatum | 19.72 | 18.62 | 16.43 | 20.81 | 21.91 |
| Sacharin | 0.01 | 0.01 | 0.02 | 0.00 | 0.00 |
| Microcrystalline Wax³ W-835 | 4.69 | 7.04 | 11.73 | 2.35 | 0.00 |

| | | | | | |
|---|---|---|---|---|---|
| * Reference formula III-E is for comparative purposes only | | | | | |

To make the above prototypes III-A-E, the wax is melted at about 95C (in Examples A-D) and the other ingredients are blended with it in a heated vessel at about 65C under vacuum. The blended products can be filled into tubes. The product can be squeezed out of the tubes onto a denture in the form of ribbons. The ribbons from products III-A-C can be picked up, and say placed at the desired location, with little or no residue on the fingers. The ribbons from product III-E cannot be picked up and placed since they are adhered to the denture surface and leave substantial residue on the fingers.

### TEST METHODS

The bioerosion of the inventive compositions can be measured by the following method: run a water source on top of the sample specimen for about 30 minutes while the specimen sits atop a wire mesh as shown in Figure 1. The water source is a laboratory faucet adjusted such that the temperature is 39 ± 1 °C and the flow rate is 16 ± 1 ml/sec. Use a funnel to focus the flow and help dampen the effect of small pressure and temperature fluctuations within the water lines. The wire mesh grid has square openings approximately 0.09 inches x 0.09 inches and is placed 2.5 inches below the tip of the funnel where it is clamped to a metal ring for support. Sample specimens weighing 0.025 g are placed on the mesh and images are taken at 0, 10 and 30 minutes to follow bio-erosion of the specimen. After 30 minutes the wire mesh containing the remainder of the specimen is removed and heated for 1 hour at 60 °C under vacuum to remove all remaining water. After the heating period, final weights are taken to calculate weight loss due to bio-erosion. An average of 3 specimens per sample is used to calculate bio-erosion time and weight loss. The composition is bioerodible if it does not leave behind visible residue, film, or sheet after about 30 minutes under these testing conditions., and/or if it cannot be easily separated or peeled away manually in one or more large pieces after 30 minutes under these testing conditions, and/or if it leaves behind less than 2, less than 4, less than 6, and/or less than 8% by weight of residue (of the original weight of the composition) after 30 minutes under these testing conditions. The above bio-erosion test may also be conducted at various time-points up to 8 hours.

The dry tack can be measured by the following method: 1. remove the article from the package material; 2. place the article on the palate-portion of a dry, acrylic upper-denture with the teeth facing downward; 3. apply pressure with fingers for 3 to 10 seconds; 4. thereafter remove finger pressure; 5. then invert the denture with the teeth facing upward. In one embodiment the article demonstrates dry tack if: i. The article does not stick to fingers during steps 1-2, ii. leaves little or no residue on the fingers in steps 3-4, and iii. in step-5, the article does not fall off of the denture, once inverted, for at least 10-30 seconds, or at least 1 minute.

In another embodiment the article demonstrates dry tack if: i. The article does not stick to fingers during steps 1-4, and ii. in step-5, the article does not fall off of the denture, once inverted, for at least 10-30 seconds, or at least 1 minute.

In another embodiment the article demonstrates dry tack if in step-5, the article does not fall off of the denture, once inverted, for at least 10-30 seconds, or at least 1 minute.

The dry tack of the inventive articles can also be measured by the following procedure:
a) Compress a 5mm diameter disc (0.67mm thick) sample of the article between a 1" diameter cylindrical probe (made from polymethylmethacrylate) and a flat sheet of polymethylmethacrylate with a 2000 gram-force for 2 seconds,
b) Pull off the probe at 1mm/second and record peak force,
c) Repeat procedure with no sample sandwiched between the two surfaces, and
d) Calculate: Dry Tack in grams/square centimeter = (Peak Force with Sample - Peak Force without sample) / Cross sectional area of sample disc.

In one embodiment the above procedure is repeated with an applied force of 250 gram-force in step-a and the tack measured in steps b-d;

The article has dry tack if the tack measured with a 250 gram-force applied force is less than 25, 50, 100, 200, or 500 grams/square-centimeter, and the tack measured with a 2000 gram-force applied force is greater than 200, 500, 1000, 2000, 5000, 10000, or 25000 grams/square-centimeter, and any combination of these levels.

The modulus G' of the inventive compositions can be measured by the following procedure:
a) Load a sample disc of 8mm diameter and 0.67mm thickness onto a ARES rheometer using a parallel plate fixture with a compressive force of 500 grams. If the sample is flowable, a sufficient amount of material is used to fill the 1 mm gap on a 25 mm diameter parallel plate fixture.
b) Set strain to be 0.02%, c. Measure G' at a sweep of frequencies including 1 Hz.

The normalized dislodgement force and dislodgement force ratio of the inventive article can be measured by the following method:
Instrument: An Instron model 5544 is used. The load cell is calibrated according to manufacturer's specifications annually. The choice of load cell is determined by having the forces generated by the adhesive fall within the recommended operating range for the load cell. This is typically between 10% - 90% of full capacity.
Test Fixtures: The geometry of a cylindrical probe and a flat plate are used as the test fixtures. The probe is made from PMMA, 0.2 sq.cm to 10 sq.cm in surface area. For the base plate, the same PMMA material is used but in sheet form, ¼" thick. This is cut into 6" x 6" plates to be clamped onto the Instron.
Hydrating Liquid: Artificial saliva containing low levels of various salts is used to hydrate the adhesive.

### Artificial Saliva Composition

| Ingredient | Amount per Liter |
|---|---|
| K₂HPO₄ | 4.2 g |
| KH₂PO₄ | 3.2 g |
| KOH | 2 pellets |
| Mineral Stock Solution | 5 ml |
| - KCl 8 g per 100 ml of Stock Solution | |
| - NaCl 8 g | |
| - Na₂SO₄ 0.264 g | |
| - MgCl₂.6H₂O 0.7687 g | |
| (or 0.36 g Anhydrous MgCl2) | |

Adhesive: 0.1 to 1.0 gram of adhesive is applied to the probe.
Hydration: The hydrating liquid (0.2 mL of artificial saliva to 2.0 ml) is pipetted onto the surface of the adhesive. The assembly is then permitted to hydrate for 20 minutes or more.
Test Method: Once the sample is hydrated, it is mounted onto the Instron and the test is carried out via computer control. The method is comprised of the following steps:
   (a) Compression to 750 to 7500 g of force
   (b) Hold at compression for 2 minutes
   (c) Reduce compressive force to 200 gf
   (d) Hold (1 minute)
   (e) Pull off at 1 mm/s
   (f) Record Peak Dislodgement Force
   (g) Calculate "Normalized Dislodgement Force" = (Peak Dislodgement Force) / (Surface Area of Probe); report in grams force per sq.cm.
   (h) Repeat steps A-F for commercial Fixodent Original denture adhesive (available commercially manufactured by P&G), or for the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.
   (i) Calculate "Dislodgement Force Ratio" = (Peak Dislodgement Force of Prototype Adhesive) / (Peak Dislodgement Force of Fixodent Original)
Data: Each sample is repeated a minimum of 3 times and the average value of the "Normalized Dislodgement Force" and "Dislodgement Force Ratio" are reported.
Specifically the normalized dislodgement force and dislodgement force ratio can be measured by using the following parameters in the procedure: 0.25 gram adhesive; 1 inch diameter probe; hydration time of 20 minutes; and compression force of 7500 grams.

The "normalized ooze amount" and "ooze ratio" of the inventive article can be measured by the following procedure:
a) Load initial sample weight of about 0.50 grams uniformly onto a 1 inch diameter cylindrical probe made from polymethylmethacrylate,
b) Bring probe to 1.2 mm of base plate, also made from polymethylmethacrylate,
c) Apply 750 gram force for 90 seconds,
d) At 90 seconds, trim and weigh material that has oozed out,
e) Calculate "Normalized Ooze Amount" = (Amount oozed out / Initial sample weight) x 100,
f) Repeat Steps a-e using commercial Fixodent Original a denture adhesive cream commercially manufactured by P&G, or with the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.,
g) Calculate "Ooze Ratio" = Normalized Ooze Amount of Prototype Adhesive / Normalized Ooze Amount of Fixodent Original,
h) Each sample is repeated a minimum of 3 times and the average value of the "Normalized Ooze Amount" and "Ooze Ratio" are reported.

## Claims

1. A denture adhesive composition in the form of a cream, paste, gel or liquid comprising a homogeneous mixture of:
a) From 10% to 90% of a denture adhesive component; and
b) From 2% to 90% of a water insoluble thermoplastic component selected from microcrystalline wax and mixtures thereof with polyethylene.

2. The composition of Claim 1 wherein the composition comprises a normalized dislodgement force of from 1100 to 12,000 grams per sq cm, or a dislodgement force ratio of from 1.1 to 10.

3. The composition of Claim 1 wherein the composition further comprises a solvent.

4. The composition of Claim 1 wherein the composition is bioerodible.

5. The composition of Claim 1 wherein the level of water insoluble thermoplastic component is from 45% to 60% by weight.

6. The composition of Claim 1 wherein the water insoluble thermoplastic component is a microcrystalline wax having a melting point of from 70°C to 90°C.

7. The composition of Claim 1 wherein the denture adhesive component is selected from cellulose derivatives, starch derivatives, saccharide derivatives, polyethylene oxides, polyethylene glycols, polyvinyl alcohol, carrageenan, alginates, karaya gum, xanthan gum, guar gum, gelatin, algin, tragacanth, chitosan, acrylamide polymers, Carbopol, polyamines, polyquaternary compounds, polyvinylpyrrolidone, AVE/MA, salts of AVE/MA, mixed salts of AVE/MA, polymeric acids, polymeric salts, polyhydroxy compounds, and mixtures thereof.

8. The composition of Claim 7 wherein the denture adhesive component comprises a salt or mixed salt of AVE/MA.

9. The composition of Claim 8 wherein the salt contains a cationic salt function comprising a cation selected from Group IA and Group 2A cations of the periodic table, yttrium, titanium, zirconium, vanadium, chromium, manganese, iron, nickel, copper, zinc, boron, aluminum, and mixtures thereof.

10. The composition of Claim 9 wherein the cation is selected from strontium, zinc, iron, magnesium, calcium, sodium, and mixtures thereof.

11. The composition of Claim 9 wherein the salt is selected from a calcium/zinc salt of AVE/MA, a magnesium/zinc/sodium salt of AVE/MA, a calcium/sodium salt of AVE/MA, a zinc salt of AVE/MA and mixtures thereof.

12. The composition of Claim 1 wherein the denture adhesive component comprises, at a level of from 5% to 60% by weight, a cellulose derivative selected from hydroxyethylcellulose, hydroxypropylmethylcellulose, sodium carboxymethylcellulose, corn starch, and mixtures thereof.

13. The composition of Claim 12 wherein the cellulose derivative is sodium carboxymethylcellulose.

14. The composition of Claim 1 further comprising a water insoluble liquid component selected from mineral oil, natural and synthetic oils, fats, silicone, silicone derivatives, dimethicone, silicone resins, hydrocarbons, hydrocarbon derivatives, essential oils, caprylic/capric triglycerides, corn, soy bean, cottonseed, castor, palm oil, coconut oil, vegetable oils, animal oils, fish oil, oleic acid, and mixtures thereof.

## Patentansprüche

1. Zahnprothesen-Haftmittelzusammensetzung in der Form einer Creme, einer Paste, eines Gels, einer Flüssigkeit, umfassend eine homogene Mischung aus:
a) von 10 % bis 90 % eines Zahnprothesen-Haftmittelbestandteils; und
b) von 2 % bis 90 % eines wasserunlöslichen thermoplastischen Bestandteils, ausgewählt aus mikrokristallinem Wachs und Mischungen davon mit Polyethylen.

2. Zusammensetzung nach Anspruch 1, wobei die Zusammensetzung eine normalisierte Dislokationskraft von 1100 bis 12.000 Gramm pro Quadratzentimeter, oder ein Dislokationskraftverhältnis von 1,1 bis 10 umfasst.

3. Zusammensetzung nach Anspruch 1, wobei die Zusammensetzung ferner ein Lösemittel umfasst.

4. Zusammensetzung nach Anspruch 1, wobei die Zusammensetzung bioerodierbar ist.

5. Zusammensetzung nach Anspruch 1, wobei der Gehalt des wasserunlöslichen thermoplastischen Bestandteils von 45 Gew.-% bis 60 Gew.-% beträgt.

6. Zusammensetzung nach Anspruch 1, wobei der wasserunlösliche thermoplastische Bestandteil ein mikrokristallines Wachs mit einem Schmelzpunkt von 70 °C bis 90 °C ist.

7. Zusammensetzung nach Anspruch 1, wobei der Zahnprothesen-Haftmittelbestandteil ausgewählt ist aus Cellulosederivaten, Stärkederivaten, Saccharid-derivaten, Polyethylenoxiden, Polyethylenglykolen, Polyvinylalkohol, Karrageen, Alginaten, Karayagummi, Xanthangummi, Guargummi, Gelatine, Algin, Traganth, Chitosan, Acrylamidpolymeren, Carbopol, Polyaminen, polyquaternären Verbindungen, Polyvinylpyrrolidon, AVE/MA, Salzen von AVE/MA, gemischten Salzen von AVE/MA, polymeren Säuren, polymeren Salzen, Polyhydroxyverbindungen, und Mischungen davon.

8. Zusammensetzung nach Anspruch 7, wobei der Zahnprothesen-Haftmittelbestandteil ein Salz oder gemischtes AVE/MA-Salz umfasst.

9. Zusammensetzung nach Anspruch 8, wobei das Salz eine kationische Salzfunktion enthält, umfassend ein Kation ausgewählt aus Gruppe IA und Gruppe IIA des Periodensystems, Yttrium, Titan, Zirkonium, Vanadium, Chrom, Mangan, Eisen, Nickel, Kupfer, Zink, Bor, Aluminium, und Mischungen davon.

10. Zusammensetzung nach Anspruch 9, wobei das Kation ausgewählt ist aus Strontium, Zink, Eisen, Magnesium, Calcium, Natrium, und Mischungen davon.

11. Zusammensetzung nach Anspruch 9, wobei das Salz ausgewählt ist aus einem Calcium/Zink-Salz von AVE/MA, einem Magnesium/Zink/Natrium-Salz von AVE/MA, einem Calcium/Natrium-Salz von AVE/MA, einem Zinksalz von AVE/MA und Mischungen davon.

12. Zusammensetzung nach Anspruch 1, wobei der Zahnprothesen-Haftmittelbestandteil, mit einem Gehalt von 5 Gew.-% bis 60 Gew.-%, ein Cellulosederivat umfasst, ausgewählt aus Hydroxyethylcellulose, Hydroxypropylmethylcellulose, Natriumcarboxymethylcellulose, Maisstärke, und Mischungen davon.

13. Zusammensetzung nach Anspruch 12, wobei das Cellulosederivat Natriumcarboxymethylcellulose ist.

14. Zusammensetzung nach Anspruch 1, ferner umfassend einen wasserunlöslichen Bestandteil, ausgewählt aus Mineralöl, natürlichen und synthetischen Ölen, Fetten, Silikonen, Silikonderivaten, Dimeticon, Silikonharzen, Kohlenwasserstoffen, Kohlenwasserstoffderivaten, ätherischen Ölen, Caprylsäure/Caprinsäuretriglyceriden, Mais, Sojabohne, Baumwollsamen, Ricinus, Palmöl, Kokosnussöl, Pflanzenölen, Tierölen, Fischöl, Oleinsäure, und Mischungen davon.

## Revendications

1. Composition adhésive pour dentier sous la forme d'une crème, d'une pâte, d'un gel ou d'un liquide comprenant un mélange homogène de :
a) 10 % à 90 % d'un composant adhésif pour dentier ; et
b) de 2 % à 90 % d'un composant thermoplastique insoluble dans l'eau choisi parmi la cire microcristalline et des mélanges de celle-ci avec du polyéthylène.

2. Composition selon la revendication 1, où la composition comprend une force normalisée de délogement allant de 1100 à 12 000 grammes par cm2, ou un rapport de force de délogement allant de 1,1 à 10.

3. Composition selon la revendication 1, où la composition comprend en outre un solvant.

4. Composition selon la revendication 1, où la composition est susceptible de bio-érosion.

5. Composition selon la revendication 1, dans laquelle le taux de composant thermoplastique insoluble dans l'eau va de 45 % à 60 % en poids.

6. Composition selon la revendication 1, dans laquelle le composant thermoplastique insoluble dans l'eau est une cire microcristalline possédant un point de fusion allant de 70 °C à 90 °C.

7. Composition selon la revendication 1, dans laquelle le composant adhésif pour dentier est choisi parmi des dérivés de cellulose, des dérivés d'amidon, des dérivés de saccharide, des oxydes de polyéthylène, des polyéthylène glycols, un alcool polyvinylique, la carraghénane, des alginates, la gomme karaya, la gomme de xanthane, la gomme de guar, la gélatine, l'algine, le tragacanthe, la chitosane, des polymères acrylamide, le Carbopol, des polyamines, des composés polyquaternaires, la polyvinylpyrrolidone, un AVE/MA (copolymère éther vinylique-acide ou anhydride maléique), des sels d'AVE/MA, des sels mixtes d'AVE/MA, des acides polymères, des sels polymères, des composés polyhydroxy, et leurs mélanges.

8. Composition selon la revendication 7, dans laquelle le composant adhésif pour dentier comprend un sel ou un sel mixte d'AVE/MA.

9. Composition selon la revendication 8, dans laquelle le sel contient une fonction sel cationique comprenant un cation choisi parmi les cations du Groupe IA et du Groupe 2A du tableau périodique, yttrium, titane, zirconium, vanadium, chrome, manganèse, fer, nickel, cuivre, zinc, bore, aluminium, et leurs mélanges.

10. Composition selon la revendication 9, dans laquelle le cation est choisi parmi le strontium, le zinc, le fer, le magnésium, le calcium, le sodium, et leurs mélanges.

11. Composition selon la revendication 9, dans laquelle le sel est choisi parmi un sel de calcium/zinc d'AVE/MA, un sel de magnésium/zinc/sodium d'AVE/MA, un sel de calcium/sodium d'AVE/MA, un sel de zinc d'AVE/MA et leurs mélanges.

12. Composition selon la revendication 1, dans laquelle le composant adhésif pour dentier comprend, à un taux allant de 5 % à 60 % en poids, un dérivé de cellulose choisi parmi l'hydroxyéthylcellulose, l'hydroxypropylméthylcellulose, la carboxyméthylcellulose de sodium, l'amidon de maïs, et leurs mélanges.

13. Composition selon la revendication 12, dans laquelle le dérivé de cellulose est la carboxyméthylcellulose de sodium.

14. Composition selon la revendication 1, comprenant en outre un composant liquide insoluble dans l'eau choisi parmi une huile minérale, des huiles naturelles et synthétiques, des graisses, de la silicone, des dérivés de silicone, de la diméthicone, des résines de silicone, des hydrocarbures, des dérivés d'hydrocarbure, des huiles essentielles, des triglycérides capriliques/capriques, du maïs, du soja, de la graine de coton, du ricin, de l'huile de palme, de l'huile de coco, des huiles végétales, des huiles animales, de l'huile de poisson, de l'acide oléique, et leurs mélanges.
